# EUROPÄISCHE PATENTSCHRIFT

(11) **EP 0 298 361 B1**
(45) Veröffentlichungstag und Bekanntmachung des Hinweises auf die Patenterteilung: **09.06.1993**
(21) Anmeldenummer: 88110393.1
(22) Anmeldetag: 29.06.1988
(51) Int. Cl.: C11D 3/42

(54) **Flüssigwaschmittel enthaltend disulfonierte optische Aufheller**
Liquid detergene containing disulfonated optical brighteners
Composition détergente liquide contenant des azurants optiques disulfonés

(30) Priorität: 03.07.1987 CH 2535/87
(43) Veröffentlichungstag der Anmeldung: 11.01.1989
(73) Patentinhaber: CIBA-GEIGY AG, 4002 Basel (CH)
(72) Erfinder: Hefti, Heinz, Dr., CH-4153 Reinach (CH); Reinehr, Dieter, Dr., D-7842 Kandern (DE); Weber, Kurt, Dr., CH-4052 Basel (CH); Eckhardt, Claude, Dr., F-68400 Riedisheim (FR)
(74) Vertreter: Schwabe - Sandmair - Marx

(56) Entgegenhaltungen:
- EP-A- 0 002 746
- CH-A- 575 461
- DE-A- 1 793 482
- DE-A- 2 808 927
- US-A- 3 904 678
- US-A- 3 940 437

## Beschreibung

Die vorliegende Anmeldung betrifft lagerstabile Flüssigwaschmittel die neben anionischen, nichtionischen oder zwitterionischen Tensiden disulfonierte Distyrylbiphenyl- bzw. Distyrylphenyl-Verbindungen als optische Aufheller enthalten, sowie neue Distyrylbiphenyl- und Distyrylphenyl-Verbindungen.

Die Verwendung von optischen Aufhellern in flüssigen Waschmitteln ist allgemein bekannt. Sie ziehen während der Behandlung auf das Waschgut auf und führen durch ihre spezielle Lichtabsorption/ Emissionseigenschaft zu einer Elimination der gelblichen Töne.

Dieser Effekt ist aber auch verantwortlich für das Auftreten von hellen Flecken wenn Textilgewebe z.B. bei einer Vorbehandlung direkt mit dem flüssigen Waschmittel in Kontakt gerät. In der EP-A-167 205 wird zur Lösung dieses Problems daher vorgeschlagen, monosulfonierte Stilbentriazolyl-, triazin- oder Distyrylbiphenyl-Aufheller zu verwenden.

Es hat sich nun überraschenderweise gezeigt, dass die Bildung von hellen Flecken, bei weiterhin ausgezeichneter Aufhellwirkung und Waschmittelstabilität, verhindert werden kann, wenn bestimmte disulfonierte Distyrylbiphenyl- bzw. Distyrylphenyl-Verbindungen als optische Aufheller in flüssige Waschmittel eingearbeitet werden.

Gegenstand der Anmeldung sind somit Flüssigwaschmittel enthaltend optische Aufheller in einer Menge von 0,01 bis 2%, dadurch gekennzeichnet, dass die optischen Aufheller aus einem Disulfonsäureaufheller oder einer Mischung von Disulfonsäureaufhellern der Formel (I) 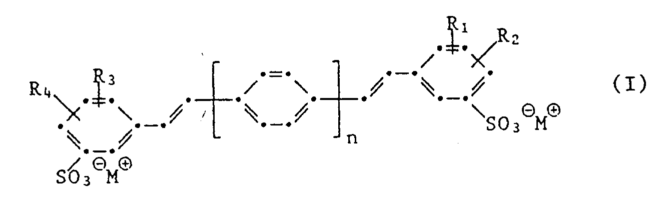
bestehen, worin
- R₁, R₂, R₃, R₄: = H, Hal und/oder C₁-C₄-Alkyl, bedeuten
- n: = 1 oder 2 bedeutet und
- M^{⊕}: - salzbildendes Kation
bedeutet, ausgenommen die Verbindungen der Formel 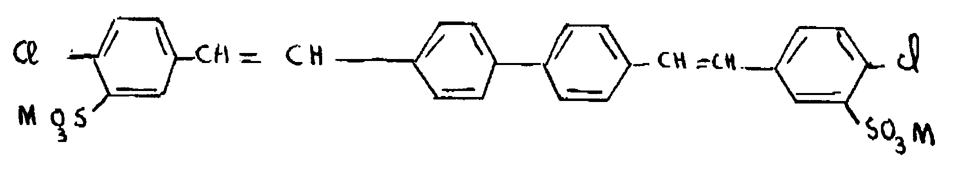
Insbesondere werden Disulfonsäureaufheller der Formel (II) 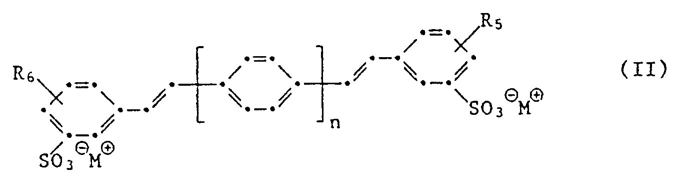
verwendet, worin
- R₅, R₆: = H, Cl, F, CH₃
- M^{⊕}: = H, Na, K, Li, NH₄, HN(CH₂-CH₂OH)₃ 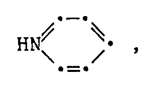 H₂N(CH₂-CH₂OH)₂,
H₃N-CH₂-CH₂OH, H₂N(CH₃)₂, H₃N-CH₃
- n: = 1 oder 2
bedeuten, ausgenommen die Verbindungen der Formel 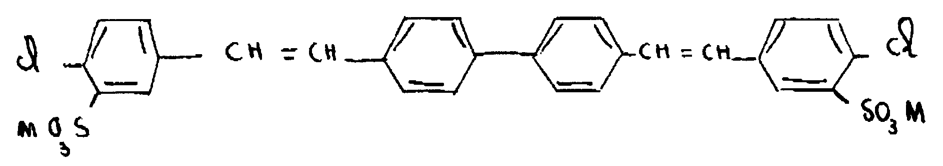
Vor allem aber werden Disulfonsäureaufheller der Formel (III) 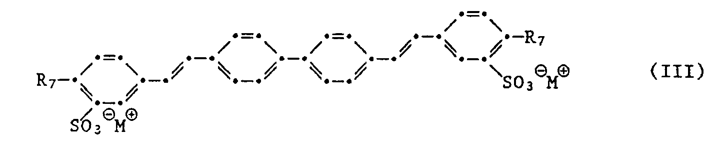
oder 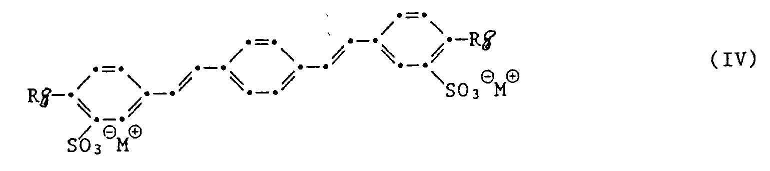
sowie Mischungen von 1 bis 10 vorzugsweise 1 bis 5 Teilen der Verbindungen der Formel III mit 10 bis 1 vorzugsweise 5 bis 1 Teilen der Verbindungen der Formel IV, worin R₇ = H, , F, CH₃ und R₈ = H, Cl, F, CH₃ ist und M^{⊕} die oben angegebene Bedeutung hat, verwendet.

Unter Flüssigwaschmitteln sind bekannte und handelsübliche Waschmittel wie sie beispielsweise in der EP-A-167 205 oder US-4 507 219 beschrieben werden, zu verstehen.

Insbesondere enthalten die Flüssigwaschmittel 1 bis 60 % anionische, nichtionische, zwitterionische und gegebenenfalls kationische Tenside und 25 bis 65 % vorzugsweise 40 bis 55 % Wasser. Im einzelnen enthält das Waschmittel neben dem optischen Aufheller 3 bis 50 % vorzugsweise 15 bis 25 % anionische Tenside, 2 bis 30 % vorzugsweise 4 bis 15 % nichtionische Tenside, 3 bis 30 % vorzugsweise 5 bis 20 % gegebenenfalls ethoxylierte (C₁₀-C₂₂)-Fettsäuren, insbesondere gesättigte (C₁₀-C₁₄)-Fettsäuren wie Kaprin-, Laurin-, Myristin-, Kokusnuss- und Palmkernsäure sowie Mischungen davon, 1 bis 25 % vorzugsweise 1 bis 10 % Waschmittelaufbaustoffe sowie gegebenenfalls 1 bis 10 % vorzugsweise 1 bis 5 % zwitterionische Tenside, 0,5 bis 3 % vorzugsweise 0,7 bis 2 % quaternäre Ammonium-, Amin- oder Aminoxid-Tenside, und 1 bis 10 % übliche Waschmittelzusätze wie zum Beispiel Enzyme, Enzymstabilisatoren, Antioxidantien, Konservierungs- und Desinfektionsmittel, Duft- und Farbstoffe, Komplexbildner bzw. Sequestriermittel und Lösungsmittel.

Brauchbare Tenside werden z.B. in der US-4 285 841, US-3 929 678, US-4 284 532 und GB-2 041 986 beschrieben. Insbesondere werden die in der EP-A-167 205 als bevorzugt bezeichneten Tenside eingesetzt. Vor allem verwendet man jedoch als anionische Tenside gegebenenfalls ethoxylierte C₁₀-C₁₈-Alkylsulfate z.B. in Form der Triethanolaminsalze, C₁₀-C₁₅-Alkylbenzolsulfonate oder Mischungen davon und als nichtionische Tenside Kondensationsprodukte aus einem Mol (C₁₀-C₁₅)-Fettalkohol mit 3 bis 8 Mol Ethylenoxid.

Als Waschmittelaufbaustoffe kommen die in der US 4 321 165 und US 4 284 532 erwähnten vorzugsweise polycarboxylierte Verbindungen wie zum Beispiel Zitronensäure oder Zitrate in Betracht.

Gegenstand der Anmeldung sind ferner die Verbindungen der Formel 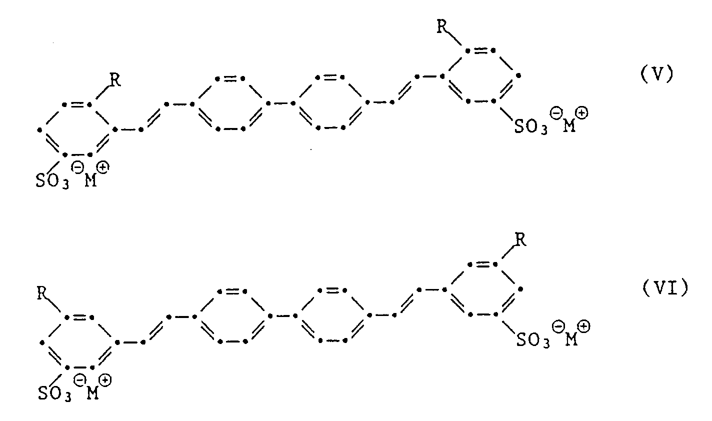
worin
- M^{⊕}: = H, Na, K, Li, NH₄, HN(CH₂-CH₂OH)₃, H₂N(CH₂-CH₂OH)₂, H₃N-CH₂-CH₂OH, H₂N(CH₃)₂, H₃N-CH₃ oder 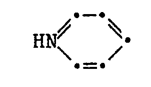
- R: = C₁-C₄-Alkyl bedeutet.

Die Verbindungen der Formel (V) und (VI) sind neu und können durch Umsetzung der Verbindung der Formel (IX) 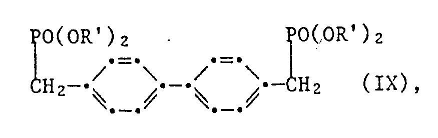
mit R' = C₁-C₄-Alkyl
in einem aprotischen Lösungsmittel wie z.B. DMSO, DMF, Acetonitril, HMPT, Benzol oder Chlorbenzol mit Verbindungen der Formeln (XI) bzw. (XII) 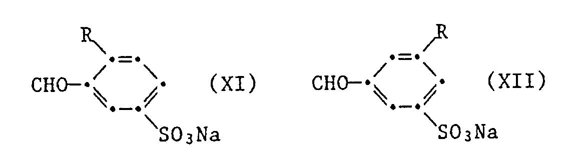
in Gegenwart von Basen wie z.B. Alkali- oder Erdalkali-Hydroxide oder -Alkoholate erhalten werden.

Die neuen Verbindungen besitzen ausgezeichnete Aufhellereigenschaften, Beständigkeit in Waschmitteln, Löslichkeit in Wasser, gutes Aufzieh- und Migrationsverhalten, hohe Aufhelleffekte auf cellulosischem Material und keine Bildung von hellen Flecken. Sie sind besonders für eine Verwendung in flüssigen Waschmitteln geeignet.

Die folgenden Beispiele dienen zur Erläuterung der Erfindung; Teile bedeuten Gewichtsteile und Prozente Gewichtsprozente; der Fleckentest wird folgendermassen durchgeführt:

### Fleckentest

a) Aufheller/Waschmittel-Formulierung:
   0,1 % (100 % Aktivsubstanz) optischer Aufheller oder Aufhellergemisch werden in einem flüssigen Waschmittel gelöst. Falls ein optischer Aufheller in der fertigen Formulierung eines flüssigen Waschmittels nicht löslich ist, wird zuerst der Aufheller in dem Wasser/Lösungsmittel- oder Tensid/Lösungsmittel-Gemisch des betreffenden Waschmittels gelöst und anschliessend werden die restlichen Waschmittelzusätze eingearbeitet.
b) Gebleichtes Baumwollgewebe wird mit der Aufheller/Waschmittel-Formulierung foulardiert. Die Flottenaufnahme beträgt 70 %. Anschliessend wird das Gewebe 15 Minuten an der Luft getrocknet.
c) 1 Teil nach b) foulardiertes Gewebe (A) und 9 Teile gebleichtes Baumwollgewebe (B) werden zusammen 15 Minuten bei 30°C ohne weitere Zusätze gewaschen (Flottenverhältnis 1:20). Anschliessend wird mit Wasser gespült und bei 70°C getrocknet.
d) Nach der Wäsche wird der Unterschied im Weissgrad (gemäss Ciba-Geigy mit einem RFC3-Photometer von Zeiss) zwischen dem foulardierten Testgewebe (A) und dem Gewebe (B) bestimmt. Ein optischer Aufheller zeigt dann eine geringe Neigung zur Bildung von hellen Flecken, wenn die Differenz zwischen dem Weissgrad des foulardierten Gewebes (A) und dem Gewebe (B) klein ist.

### Beispiel 1: Der Fleckentest wird mit optischen Aufhellern der Formel

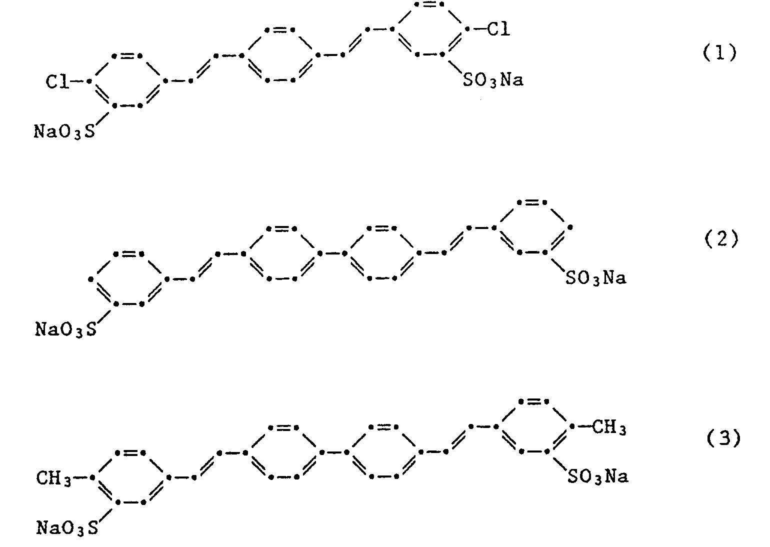

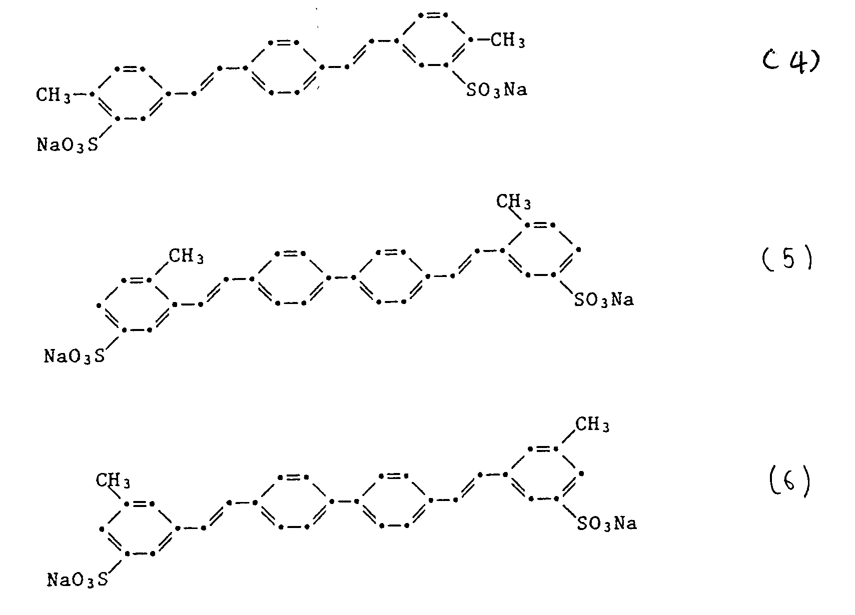

sowie mit folgenden Mischungen

| | |
|---|---|
| (20) | 4 Teile Verbindung (1) mit 1 Teil Verbindung (3) |
| (21) | 1 Teil Verbindung (1) mit 1 Teil Verbindung (3) |

und einem flüssigen Waschmittel enthaltend
15 % C₁₁-C₁₃ Alkylbenzolsulfonat
14 % C₁₄-C₁₅-Polyethoxyfettalkohol (Ethylenoxid 7)
10 % Seifenflocken
9 % Ethanol
4 % Na-Citrat
5 % Triethanolamin
43 % Wasser
durchgeführt. Alle Verbindungen sind über mehrere Monate im Waschmittel beständig und zeigen gute Aufhellwirkung und keine oder lediglich geringe Bildung von hellen Flecken.

### Beispiel 2: Der Fleckentest wird mit Verbindungen der Formel (1)-(6) sowie den Mischungen (20) oder (21) und einem flüssigen Waschmittel enthaltend

7,5 % C₁₃ Alkylbenzolsulfonat
12 % C₁₄-C₁₅ Alkylpolyethoxysulfonat (Ethylenoxid 2.25)
15 % C₁₁-C₁₃ Fettsäure-Kaliumsalz
10 % C₁₂-C₁₃ Polyethoxyfettalkohol (Ethylenoxid 8)
5,5 % Na-Citrat
12 % 1:1 Mischung von Isopropylalkohol und Spiritus
0,7 % C₁₂ Alkyltrimethylammoniumchlorid
37,3 % Wasser
durchgeführt. Alle Verbindungen sind über mehrere Monate im Waschmittel beständig und zeigen gute Aufhellwirkung und keine oder lediglich geringe Bildung von Flecken.

### Beispiel 3: Der Fleckentest wird mit Verbindungen der Formel (1)-(6) sowie den Mischungen (20) oder (21) und einem flüssigen Waschmittel enthaltend

11,5 % C₁₁-C₁₃ Alkylbenzolsulfonat
3,8 % Triethanolaminlaurylsulfat
15,5 % Kalium-Seife
15 % C₁₄-C₁₅-Polyethoxyfettalkohol (Ethylenoxid 7)
5 % Triethanolamin
10 % Ethanol
39,2 % Wasser
durchgeführt. Alle Verbindungen sind über mehrere Monate im Waschmittel beständig und zeigen gute Aufhelleigenschaften und keine oder lediglich geringe Bildung von Bleichflecken.

### Beispiel 4:

a) 10,2 g Natrium-methylat (Gehalt: 96 %) werden unter Rühren in 100 ml Dimethylformamid vorgelegt. Dazu wird eine Lösung von 31,7 g 4,4'-Bis-(dimethylphosphonomethyl)-biphenyl (Gehalt: 94,3 %) und 33,3 g 2-Methylbenzaldehyd-5-sulfosäure Natriumsalz in 600 ml Dimethylformamid bei 40-45°C innert 3 Stunden zugetropft. Nach Zugabe von 100 ml Wasser wird mit 5 ml konz. Salzsäure neutralisiert, zur Trockene eingedampft und der Rückstand aus einer Mischung von 200 ml Wasser und 600 ml Ethanol umkristallisiert. Man erhält so 17,7 g der Verbindung der Formel (5).
b) 2-Methylbenzaldehyd-5-sulfosäure, Natriumsalz kann wie folgt erhalten werden:
   120 g 2-Methylbenzaldehyd werden unter Rühren innert 1 1/2 Stunden in 300 ml Oleum 66 % eingetropft, wobei die Temperatur auf 49°C ansteigt. Das Reaktionsgemisch wird auf Raumtemperatur abgekühlt, auf 1,5 kg Eis gegossen und mit 1450 g Bariumcarbonat neutralisiert. Das Bariumsulfat wird abgenutscht, mit Wasser gewaschen und das Filtrat am Rotationsverdampfer auf 800 ml eingeengt. Nach Abkühlen auf 0-5°C wird das auskristallisierte Bariumsalz abgenutscht, in 1000 ml Wasser heiss gelöst und tropfenweise mit 22,5 g Schwefelsäure (Gehalt: 95,7 %) versetzt. Die Ausfällung von Bariumsulfat wird heiss abgenutscht, das Filtrat mit 2n Natronlauge neutralisiert und zur Trockene eingedampft. Man erhält 91 g 2-Methylbenzaldehyd-5-sulfosäure Natriumsalz.
c) Auf analoge Weise wie in Abschnitt a) dieses Beispiels beschrieben, kann unter Verwendung von 3-Methylbenzaldehyd-5-sulfosäure Natriumsalz die Verbindung der Formel (6) erhalten werden.

3-Methylbenzaldehyd-5-sulfosäure Natriumsalz kann wie im Beispiel 3b) beschrieben durch Sulfierung von 3-Methylbenzaldehyd mit Oleum 66 % und Isolierung über das Bariumsalz erhalten werden.

## Patentansprüche

1. Flüssigwaschmittel enthaltend optische Aufheller in einer Menge von 0,01 bis 2%, dadurch gekennzeichnet, dass die optischen Aufheller aus einem Disulfonsäureaufheller oder einer Mischung von Disulfonsäureaufhellern der Formel (I) 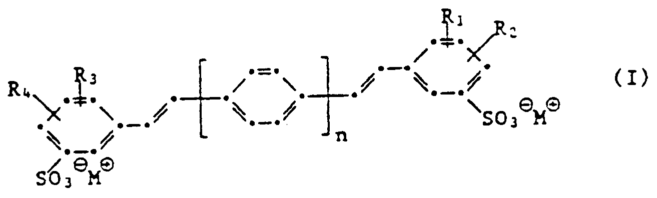 bestehen, worin
R₁, R₂, R₃, R₄ = H, Hal und/oder C₁-C₄-Alkyl bedeuten,
n = 1 oder 2 bedeutet und
M^{⊕} = salzbildendes Kation
bedeutet, ausgenommen die Verbindungen der Formel

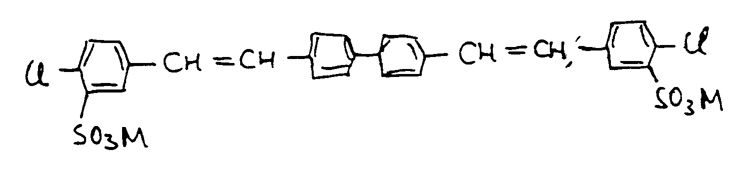

2. Flüssigwaschmittel gemäss Anspruch 1, dadurch gekennzeichnet, dass das Flüssigwaschmittel Disulfonsäureaufheller oder eine Mischung von Disulfonsäureaufhellern der Formel (II) 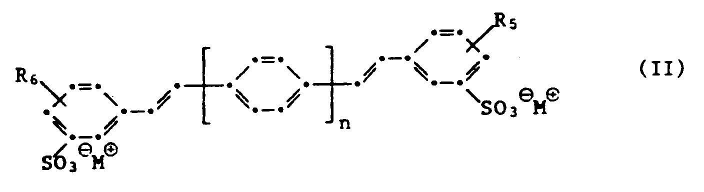 enthält, worin
R₅, R₆ = H, Cl, F, CH₃,
M^{⊕} = H, Na, K, Li, NH₄, HN(CH₂-CH₂OH)₃ 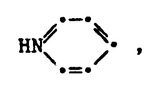 H₂N(CH₂-CH₂OH)₂, H₃N-CH₂-CH₂OH, H₂N(CH₃)₂, H₃N-CH₃ und
n = 1 oder 2
bedeuten, ausgenommen die Verbindungen der Formel

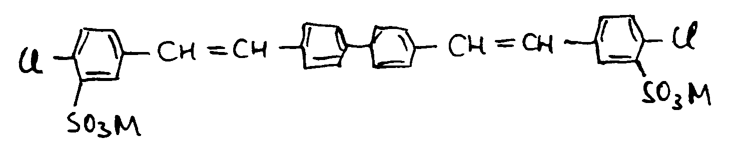

3. Flüssigwaschmittel gemäss Anspruch 2, dadurch gekennzeichnet, dass das Flüssigwaschmittel Disulfonsäureaufheller der Formel (III) 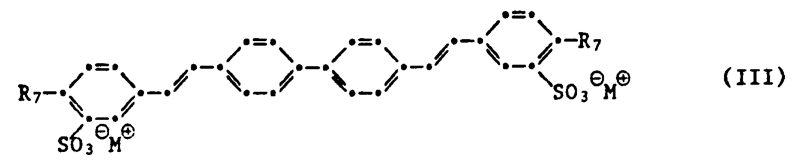 enthält, worin R₇ = H, , F, CH₃ ist und M^{⊕} die in Anspruch 2 genannte Bedeutung hat.

4. Flüssigwaschmittel gemäss Anspruch 2, dadurch gekennzeichnet, dass das Flüssigwaschmittel Disulfonsäureaufheller der Formel (IV) 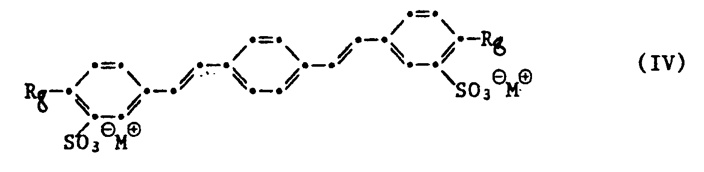 enthält, worin R₈ = H, Cl, F, CH₃ ist und M^{⊕} die in Anspruch 2 genannte Bedeutung hat.

5. Flüssigwaschmittel gemäss Anspruch 2, dadurch gekennzeichnet, dass das Flüssigwaschmittel eine Mischung von 1 bis 10 Teilen Disulfonsäureaufhellern der Formel (III) 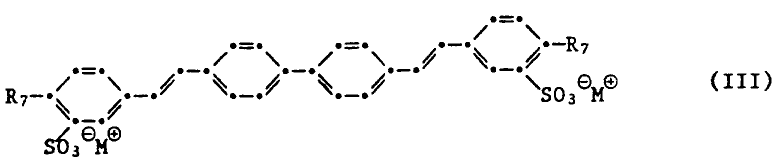 mit 10 bis 1 Teilen Disulfonsäureaufhellern der Formel (IV) 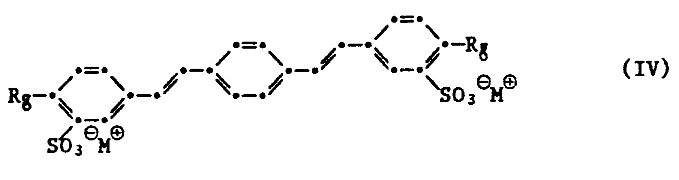 enthält, worin R₇ = H, , F, CH₃ und R₈ = H, Cl, F und CH₃ ist und M^{⊕} die in Anspruch 2 genannte Bedeutung hat,
ausgenommen die Verbindungen der Formel

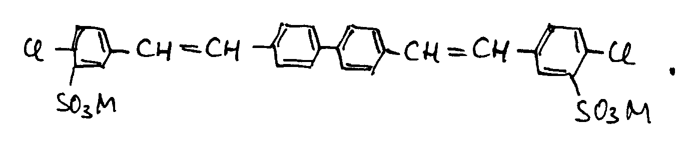

6. Flüssigwaschmittel gemäss einem der Ansprüche 1-5, dadurch gekennzeichnet, dass das Waschmittel 25 % bis 65 % Wasser, 3 % bis 50 % anionische Tenside, 2 % bis 30 % nichtionische Tenside, 3 % bis 30 % (C₁₀-C₂₂)-Fettsäuren, 1 bis 10 % Waschmittelaufbaustoffe und gegebenenfalls weitere Waschmittelzusätze enthält.

7. Verbindungen der Formel (V) 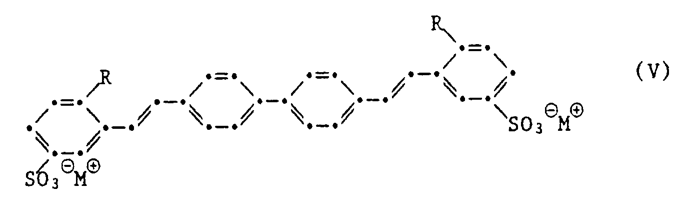
M^{⊕} = H, Na, K, Li, NH₄, HN(CH₂-CH₂OH)₃, H₂N(CH₂-CH₂OH)₂, worin H₃N-CH₂-CH₂OH, H₂N(CH₃)₂, H₃N-CH₃ oder 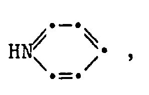 und
R = C₁-C₄-Alkyl bedeutet.

8. Verbindungen der Formel (VI) 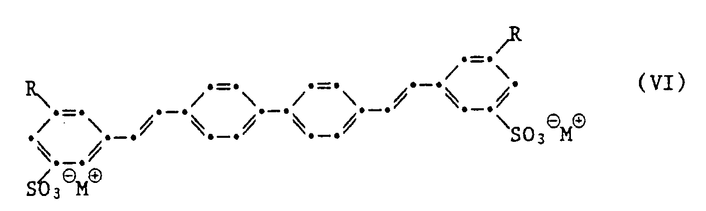 worin
M^{⊕} = H, Na, K, Li, NH₄, HN(CH₂-CH₂OH)₃, H₂N(CH₂-CH₂OH)₂, H₃N-CH₂-CH₂OH, H₂N(CH₃)₂, H₃N-CH₃ oder 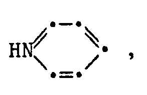 und
R = C₁-C₄-Alkyl bedeutet.

9. Verfahren zur Herstellung der Verbindungen der Formel (V) und (VI), , dadurch gekennzeichnet, dass man die Verbindung der Formel (IX) 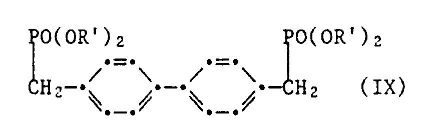 mit R' = C₁-C₄-Alkyl
in einem aprotischen Lösungsmittel mit Verbindungen der Formeln (XI) bzw. (XII) 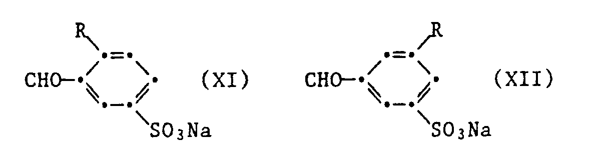 in Gegenwart einer Base umsetzt und gegebenenfalls anschliessend in die freie Säure bzw. Salze überführt.

10. Verwendung der Verbindungen der Formel (V) und (VI) in Waschmitteln zum optischen Aufhellen von cellulosischen Textilgeweben.

## Claims

1. A liquid detergent composition containing fluorescent whitening agents in an amount of 0.01 to 2 %, wherein the fluorescent whitening agents consist of a disulfonic acid whitening agent or a mixture of disulfonic acid whitening agents of the formula (I) 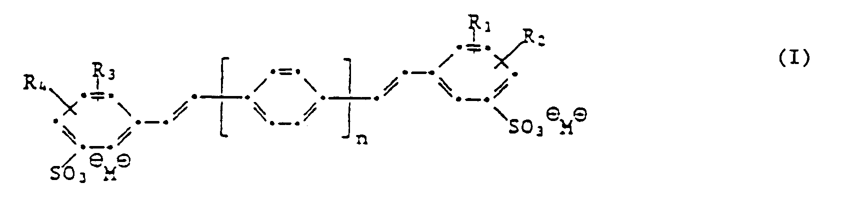 in which
R₁, R₂, R₃, R₄ = H, Hal and/or C₁-C₄alkyl,
n = 1 or 2 and
M^{⊕} = a salt-forming cation.
with the exception of the compounds of the formula

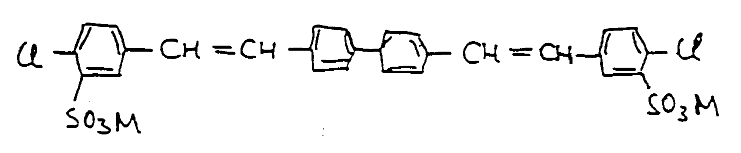

2. A liquid detergent composition according to claim 1, which contains disulfonic acid whitening agents or a mixture of disulfonic acid whitening agents of the formula (II) 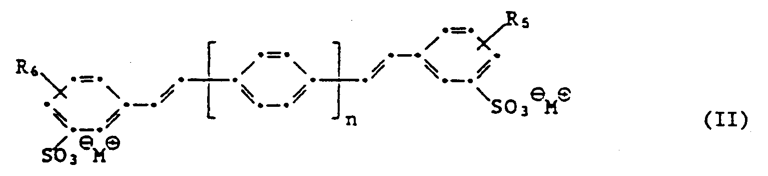 in which
R₅, R₆ = H, Cl, F, CH₃
M^{⊕} = H, Na, K, Li, NH₄, HN(CH₂-CH₂OH)₃, 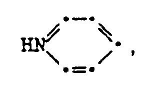 H₂N(CH₂-CH₂OH)₂ H₃N-CH₂-CH₂OH, H₂N(CH₃)₂ or H₃N-CH₃ and
n = 1 or 2,
with the exception of the compounds of the formula

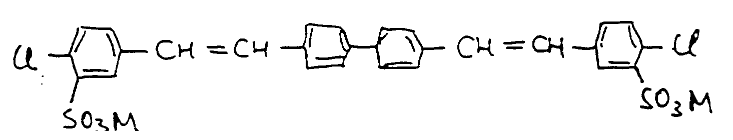

3. A liquid detergent composition according to claim 2, which contains disulfonic acid whitening agents of the formula (III) 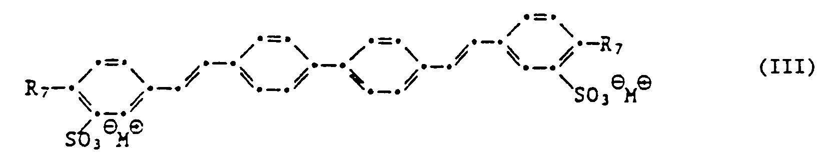 in which R₇ = H, F or CH₃ and M^{⊕} is as defined in claim 2.

4. A liquid detergent composition according to claim 2, which contains disulfonic acid whitening agents of the formula (IV) 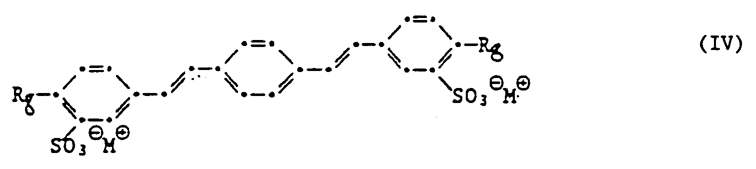 in which R₈ = H, Cl, F or CH₃ and M^{⊕} is as defined in claim 2.

5. A liquid detergent composition according to claim 2, which contains a mixture of 1 to 10 parts of disulfonic acid whitening agents of the formula (III) 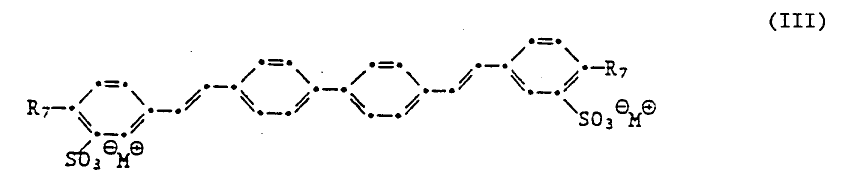 with 10 to 1 parts of disulfonic acid whitening agents of the formula (IV) 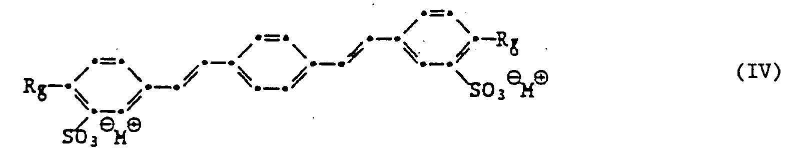 in which R₇ = H, F or CH₃ and R₈ = H, Cl, F or CH₃ and M^{⊕} is as defined in claim 2, with the exception of the compounds of the formula

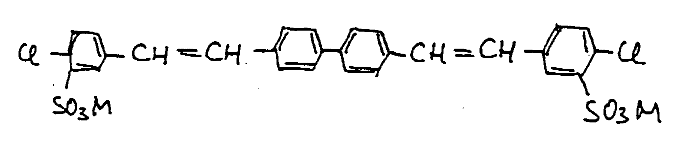

6. A liquid detergent composition according to any one of claims 1-5, which contains 25 % to 65 % of water, 3 % to 50 % of anionic surfactants, 2 % to 30 % of nonionic surfactants, 3 % to 30 % of (C₁₀-C₂₂) fatty acids, 1 to 10 % of detergent builders and, if appropriate, other detergent additives.

7. A compound of the formula (V) 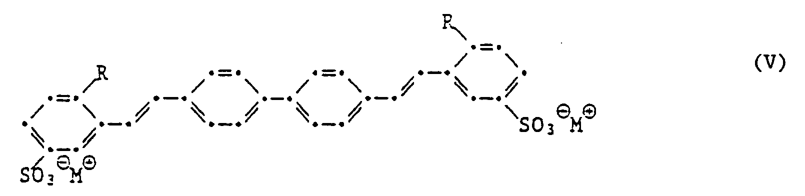 in which
M^{⊕} = H, Na, K, Li, NH₄, HN(CH₂-CH₂OH)₃, H₂N(CH₂-CH₂OH)₂, H₃N-CH₂-CH₂OH, H₂N(CH₃)₂, H₃N-CH₃ or 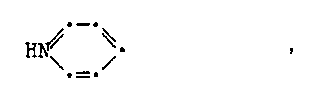 and
R = C₁-C₄alkyl.

8. A compound of the formula (VI) 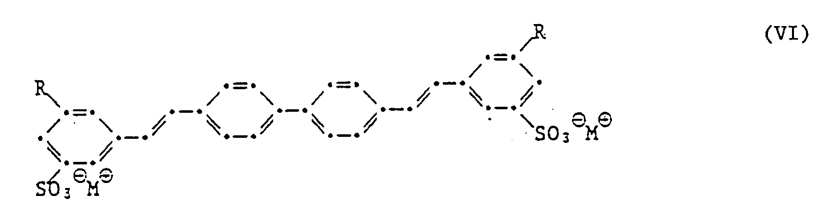 in which
M^{⊕} = H, Na, K, Li, NH₄, HN(CH₂-CH₂OH)₃, H₂N(CH₂-CH₂OH)₂, H₃H-CH₂-CH₂OH, H₂N(CH₃)₂, H₃N-CH₃ or 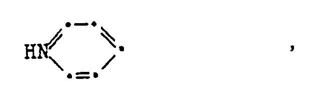 and
R = C₁-C₄alkyl.

9. A process for the preparation of a compound of the formula (V) or (VI), which comprises reacting a compound of the formula (IX) 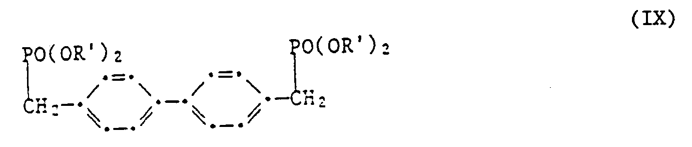 where
R' = C₁-C₄alkyl
in an aprotic solvent, with a compound of the formula (XI) or (XII) 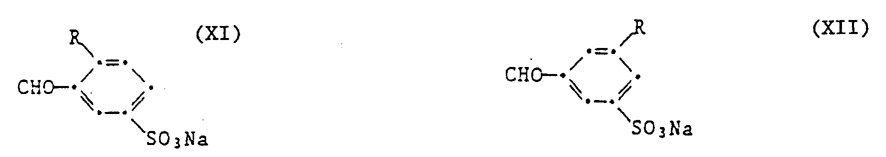 in the presence of a base, and, if desired, subsequently converting the product into the free acid or a salt.

10. Use of a compound of the formula (V) or (VI) in detergents for the fluorescent whitening of cellulosic textile fabrics.

## Revendications

1. Détergent liquide contenant des azurants optiques, en une proportion de 0,01 à 2 %, et caractérisé en ce que ces azurants optiques sont constitués d'un azurant disulfoné ou d'un mélange d'azurants disulfonés de formule (I) : 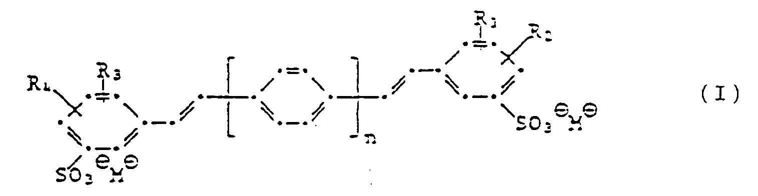 dans laquelle R₁, R₂, R₃ et R₄ représentent des atomes d'hydrogène ou d'halogène et/ou des groupes alkyle en C₁-C₄, n est un nombre valant 1 ou 2 et M⁺ représente un cation formant un sel,
à l'exception des composés de formule :

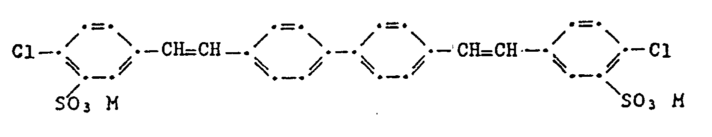

2. Détergent liquide conforme à la revendication 1, caractérisé en ce qu'il contient un azurant disulfoné ou un mélange d'azurants disulfonés de formule (II) : 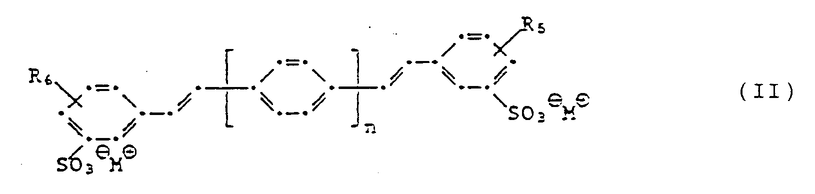 dans laquelle :
R₅, R₆ = H, Cl, F, CH₃
M⁺ = H, Na, K, Li, NH₄, HN(CH₂-CH₂OH)₃, 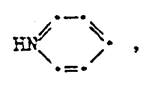 H₂N(CH₂-CH₂OH)₂, H₃N-CH₂-CH₂OH, H₂N(CH₃)₂, H₃N-CH₃
n = 1 ou 2
à l'exception des composés de formule :

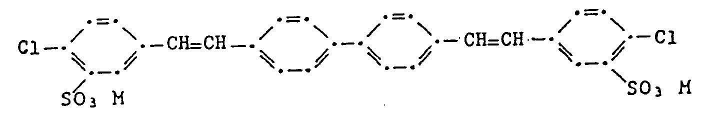

3. Détergent liquide conforme à la revendication 2, caractérisé en ce qu'il contient un azurant disulfoné de formule (III) : 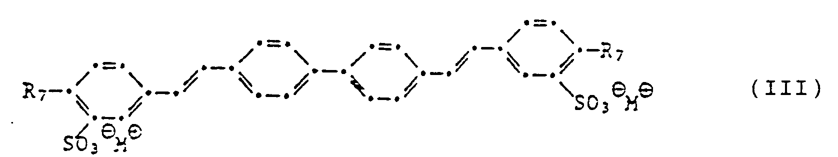 dans laquelle R₇ représente un atome d'hydrogène ou de fluor ou un groupe méthyle et M⁺ a la signification indiquée dans la revendication 2.

4. Détergent liquide conforme à la revendication 2, caractérisé en ce qu'il contient un azurant disulfoné de formule (IV) : 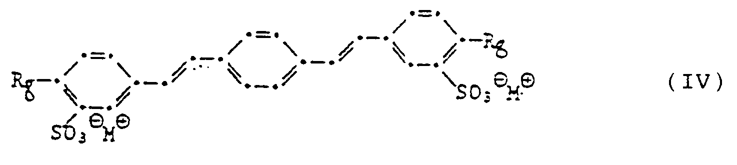 dans laquelle R₈ représente un atome d'hydrogène, de chlore ou de fluor ou un groupe méthyle et M⁺ a la signification indiquée dans la revendication 2.

5. Détergent liquide conforme à la revendication 2, caractérisé en ce qu'il contient un mélange de 1 à 10 parties d'un azurant disulfoné de formule (III) : 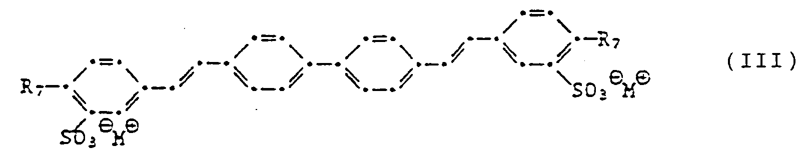 et de 10 à 1 parties d'un azurant disulfoné de formule (IV) : 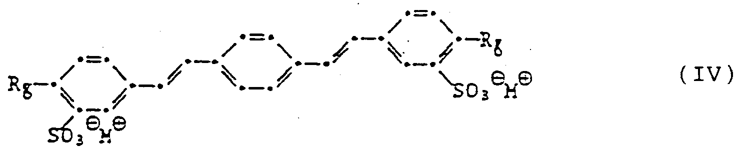 dans lesquelles R₇ représente un atome d'hydrogène ou de fluor ou un groupe méthyle, R₈ représente un atome d'hydrogène, de chlore ou de fluor ou un groupe méthyle et M⁺ a la signification indiquée dans la revendication 2,
à l'exception du composé de formule :

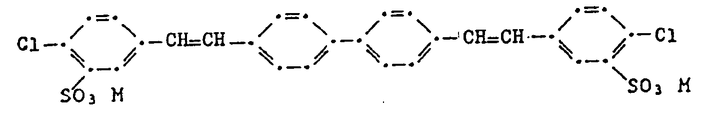

6. Détergent liquide conforme à l'une quelconque des revendications 1 à 5, caractérisé en ce qu'il contient de 25 % à 65 % d'eau, de 3 % à 50 % de tensioactifs anioniques, de 2 % à 30 % de tensioactifs nonioniques, de 3 % à 30 % d'acides gras en C₁₀-C₂₂, de 1 % à 10 % d'adjuvants actifs pour détergents, et éventuellement d'autres adjuvants pour détergents.

7. Composés de formule (V) : 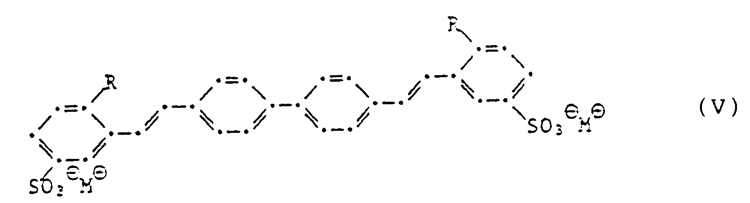 dans laquelle M⁺ représente H, Na, K, Li, NH₄, HN(CH₂-CH₂OH)₃, H₂N(CH₂-CH₂OH)₂, H₃N-CH₂-CH₂OH, H₂N(CH₃)₂, H₃N-CH₃ ou 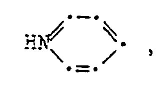 et R représente un groupe alkyle en C₁-C₄.

8. Composés de formule (VI) : 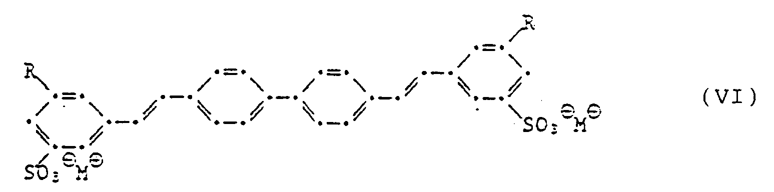 dans laquelle M⁺ représente H, Na, K, Li, NH₄, HN(CH₂-CH₂OH)₃, H₂N(CH₂-CH₂OH)₂, H₃N-CH₂-CH₂OH, H₂N(CH₃)₂, H₃N-CH₃ ou 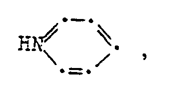 et R représente un groupe alkyle en C₁-C₄.

9. Procédé de préparation des composés de formules (V) et (VI), caractérisé en ce que l'on fait réagir le composé de formule (IX) : 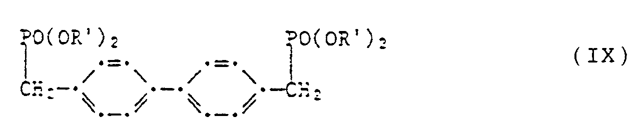 dans laquelle R' représente un groupe alkyle en C₁-C₄, dans un solvant aprotique, avec un composé de formule (XI) ou (XII) : 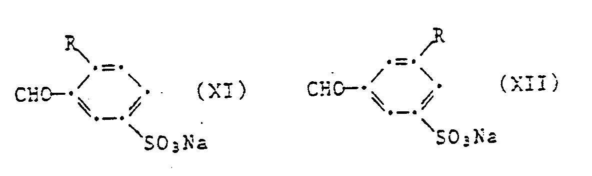 en présence d'une base, et que l'on convertit ensuite, éventuellement, le produit obtenu en acide libre ou en sel.

10. Utilisation des composés de formules (V) et (VI) dans des détergents destinés à l'azurage optique de tissus textiles cellulosiques.
